# EUROPÄISCHE PATENTSCHRIFT

(11) **EP 2 790 787 B1**
(45) Veröffentlichungstag und Bekanntmachung des Hinweises auf die Patenterteilung: **04.01.2017**
(21) Anmeldenummer: 12783226.9
(22) Anmeldetag: 09.11.2012
(51) Int. Cl.: A61Q 5/06, A61K 8/02, A61K 8/25, A61K 8/26, A61K 8/29, A61K 8/34

(54) **PULVERFÖRMIGE HAARKOSMETIKA**
POWDERY HAIR COSMETICS
PRODUITS COSMÉTIQUES CAPILLAIRES PULVÉRULENTS

(30) Priorität: 16.12.2011 DE 102011088840
(43) Veröffentlichungstag der Anmeldung: 22.10.2014
(73) Patentinhaber: Henkel AG & Co. KGaA, 40589 Düsseldorf (DE)
(72) Erfinder: KNAPPE, Thorsten, 22869 Schenefeld (DE); HENSCHEL, Anna, 21423 Winsen (DE)
(86) Internationale Anmeldenummer: PCT/EP2012/072236
(87) Internationale Veröffentlichungsnummer: WO 2013/087311

(56) Entgegenhaltungen:
- WO-A1-2006/056377
- WO-A1-2007/051511
- WO-A1-2009/120526
- WO-A1-2010/054980
- WO-A1-2012/168073
- WO-A2-2010/115700

## Beschreibung

Die vorliegende Erfindung betrifft das technische Gebiet der temporären Umformung keratinhaltiger Fasern, insbesondere das technische Gebiet der temporären Haarumformung.

Stylingmittel zur Verformung keratinhaltiger Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.

Entsprechende Mittel zur temporären Formgebung enthalten als formgebende Komponente üblicherweise synthetische Polymere. Zubereitungen, die ein Polymer enthalten, können mittels Treibgasen oder durch einen Pumpmechanismus auf das Haar aufgebracht werden. Haargele und Haarwachse werden hingegen in der Regel nicht direkt auf das Haar appliziert, sondern mittels eines Kamms oder der Hände im Haar verteilt.

Bekannte Formen temporärer Stylingmittel lassen sich oftmals nicht mit zufriedenstellender Genauigkeit dosieren. So lassen sich etwa Haargele, Haarcremes und Haarwachse nur noch schwierig verteilen, sobald sie einmal auf das Haar aufgebracht sind. Sobald der Kamm oder die Hände, auf die das Stylingmittel aufgebracht wurde, mit den ersten Haarpartien in Kontakt kommen, werden vergleichsweise große Mengen an Stylingmittel an das Haar abgegeben. In Haarpartien, die erst später mit dem Kamm oder den Händen erreicht werden, wird hingegen vergleichsweise wenig Stylingmittel eingearbeitet. Dies hat zur Folge, dass der Anwender entweder von Anfang an eine große Menge Stylingmittel aufbringen muss, so dass auch die zuletzt erreichten Haarpartien ausreichend Stylingmittel erhalten, oder gezwungen ist, das Stylingmittel in mehreren Schritten aufzubringen, wobei jeweils andere Haarpartien behandelt werden. Haarsprays lassen sich gleichmäßiger auf das Haar verteilen. Da der Verwender aber keine Möglichkeit hat, die Gesamtmenge an aufgebrachtem Stylingmittel visuell zu erfassen, besteht die Gefahr, dass mehr Stylingmittel auf das Haar aufgebracht wird, als eigentlich erforderlich wäre.

Pulverförmige Kosmetika sind bekannt und werden etwa im Bereich der Hautbehandlung bereits seit langem eingesetzt. Typische Beispiele sind etwa Make-up Puder oder Lidschatten. Um die pulverförmige Konsistenz zu erzielen, ist der Einsatz eines pulverförmigen Trägermaterials erforderlich. Als geeignetes Trägermaterial kann etwa ein Metalloxid, wie z.B. Siliziumdioxid verwendet werden. Besonders interessant ist hydrophobiertes Metalloxid bzw. Siliziumdioxid. Dieses kann beispielsweise ausgehend von pyrogenem Siliziumdioxid, das in verschiedenen Spezifikationen kommerziell erhältlich ist, erhalten werden. Auf der Oberfläche trägt unbehandeltes pyrogenes Siliziumdioxid Silanol- und Siloxangruppen. Dadurch hat es eine hohe Affinität zu Wasser, d.h. es ist hydrophil. Durch Umsetzung mit geeigneten organischen Siliziumverbindungen lassen sich Alkylsilylgruppen auf der Oberfläche des pyrogenen Siliziumdioxids chemisch binden. Es entstehen modifizierte Siliziumdioxidpulver, die nicht mehr von Wasser benetzt werden, d.h. die hydrophobe Eigenschaften aufweisen.

Der Einsatz von hydrophobiertem Siliziumdioxid in der Kosmetik zur Herstellung von so genanntem trockenen Wasser für die Haut ist dem Fachmann bekannt. Dabei werden die hydrophoben Eigenschaften des modifizierten Siliziumdioxids ausgenutzt, die bewirken, dass das Siliziumdioxid beim intensiven Mischen mit Wasser nicht einfach in diesem dispergiert wird. Die Wassertropfen werden vielmehr von den hydrophoben Feststoffpartikeln umhüllt und am erneuten Zusammenfließen gehindert. Auf diese Weise lassen sich pulverförmige Feststoffe mit einem Wassergehalt von bis zu über 95 % erhalten. Unter mechanischer Beanspruchung, beispielsweise beim Verreiben auf der Haut, wird das eingeschlossene Wasser wieder freigesetzt. Dieses trockene Wasser wird als Grundlage zur Herstellung von lagerstabilem, festem Wasserstoffperoxid und von verstreichbaren Zubereitungen mit sehr geringem Ölgehalt beschrieben.

Dieses Konzept liegt auch der in EP 1 235 554 B1 beschriebenen Herstellung kosmetischer oder pharmazeutischer, verflüssigbarer Pulverzusammensetzungen zu Grunde. Die Pulverzusammensetzungen umfassen hydrophob beschichtete Siliziumdioxidteilchen, in die Wasser und ein wasserlösliches Polymer eingeschlossen sind, wobei die Zusammensetzungen weniger als 1 % Öl enthalten. Durch Zugabe des wasserlöslichen Polymers soll erreicht werden, dass sich das Pulver bei der Anwendung auf der Haut angenehm und nicht körnig anfühlt, ohne dass zu diesem Zweck dem Produkt eine Ölkomponente zugegeben werden müsste. Das Polymer wird zu diesem Zweck der Wasserphase in einer Menge von 0,01 bis 5 Gew.-% zugegeben, wobei ein Gehalt an lediglich 0,1 bis 1 Gew.-% bevorzugt ist. Die verflüssigbaren Pulverzusammensetzungen werden vor allem zur Herstellung dekorativer Kosmetika eingesetzt. Daneben sind auch der Einsatz in Deodorants oder Sonnenschutzmitteln, oder die Anwendung auf dem Haar als Basis von Haarbehandlungsmitteln, die Perlglanzmittel oder Pflegekomponenten enthalten, beschrieben. Eine Verwendung im Bereich der Stylingmittel ist nicht genannt.

WO 03/037287 A1 offenbart die Verwendung eines Granulats auf Basis von pyrogenem Siliziumdioxid in kosmetischen Zusammensetzungen. Die speziellen Granulate können silanisiert, d.h. hydrophobiert werden und eignen sich zur Herstellung kosmetischer Zusammensetzungen jeglicher Konsistenz, beispielsweise Flüssigkeiten, Schäume, Sprays oder Pulver. Als mögliche kosmetische Zusammensetzungen werden eine Vielzahl denkbarer Kosmetika, unter anderem Haarstylingmittel, genannt. Dabei werden jedoch nur die üblichen Applikationsformen Lotion, Haarspray, Haarlack, Haargel und Haarwachs genannt. Ein Hinweis darauf, dass auf Basis des beschriebenen Siliziumdioxids pulverförmige Stylingmittel hergestellt werden könnten, findet sich nicht.

Die Druckschrift WO 2007/051511 A1 offenbart die Verwendung einer pulverförmigen Zusammensetzung, enthaltend 50 bis 95 Gew.-% eines wässrigen Lösungsmittels, hydrophobiertes Siliziumdioxidpulver und mindestens im wässrigen Lösemittel befindliches filmbildendes und/oder festigendes Polymer zur temporären Verformung keratinischer Fasern.

Die Druckschrift DE102008057261 A1 betrifft pulverförmige Zusammensetzungen zur temporären Umformung von Haaren für sehr starkem Halt der fixierten Frisur. Besagte Pulver umfassen Kern-Hülle-Teilchen, deren Hülle Partikel mindestens eines hydrophobierten Metalloxidpulvers enthält und deren Kern eine flüssige, wässrige Phase umfasst, dadurch gekennzeichnet, dass die pulverförmige Zusammensetzung mindestens ein filmbildendes und/oder festigendes Polymer in Form von Partikeln enthält.

Die internationale Patentanmeldung WO 2010/115700 A2 beschreibt pulverförmige Zusammensetzungen zur temporären Verformung menschlicher Haare, die neben weiteren Bestandteilen, bezogen auf ihr Gesamtgewicht 50 bis 90 Gew.-% Wasser und 0,5 bis 10 Gew.-% Glycerin enthalten.

Die pulverförmigen Haarkosmetika des Standes der Technik liefen mittlerweile zwar einen für die Haarumformung akzeptablen Halt. Allerdings ist das mit diesen Mitteln erzielte Ergebnis bezüglich der Parameter natürlicher Glanz sowie der Elastizität des Halts verbesserungswürdig. Zudem sind die meisten üblichen Stylingrohstoffe wie Wachse, Öle oder Polymere nicht ohne weiteres zur Herstellung stabiler pulverförmiger Zusammensetzungen geeignet. Entweder verhindern sie die erfolgreiche Bildung der Kern-Hülle-Teilchen, oder die Lagerstabilität der gebildeten Kern-Hülle-Teilchen wird herabgesenkt.

Aufgabe der vorliegenden Erfindung war es daher, ein pulverförmiges Haarbehandlungsmittel zur temporären Formgebung zur Verfügung zu stellen, das
- genau und einfach dosiert werden kann,
- lagerstabil ist,
- das Haar nicht verklebt,
- dem Haar einen volleren sowie natürlichen Griff verleiht,
- dem Haar natürlichen Glanz verleiht.

Die Haltbarkeit des Stylingergebnisses soll dabei nicht beeinträchtigt werden.

Es wurde gefunden, dass die Lehre des Standes der Technik durch eine nachfolgend beschriebene pulverförmige Zusammensetzung verbessert wird, die im flüssigen Kern ein spezielles Polyol in einer speziellen Menge erhält.

Ein erster Gegenstand der vorliegenden Erfindung sind pulverförmige Zusammensetzungen zur temporären Umformung keratinhaltiger Fasern, insbesondere menschlicher Haare, umfassend

Kern-Hülle-Teilchen, deren Hülle Partikel mindestens eines hydrophobierten Metalloxidpulvers enthält und deren flüssiger Kern bezogen auf das Gewicht des Kerns 20,0 Gew.-% bis 100,0 Gew.-%, insbesondere 50,0 Gew.-% bis 100,0 Gew.-%, Glycerin enthält, dadurch gekennzeichnet, dass zusätzlich mindestens ein kationisches, amphoteres oder anionisches festigendes Polymer enthalten ist.

Unter keratinhaltigen Fasern sind dabei erfindungsgemäß Pelze, Wolle, Federn und insbesondere menschliche Haare zu verstehen.

Die Kern-Hülle-Teilchen der pulverförmigen Zusammensetzung gemäß vorliegender Erfindung umfassen einen flüssigen Kern. Diesen flüssigen Kern umgibt eine Hülle, die auf separierbaren einzelnen Partikeln mindestens eines hydrophobierten Metalloxidpulvers basiert.

Partikel sind im Sinne der Erfindung als Korn vorliegende Teilchen (vgl. DIN 66160: 1992-09) von Festkörpern.

Pulverförmig im Sinne der Erfindung sind Zusammensetzungen, deren Teilchen unter eigenem Gewicht frei rieselfähig sind (vgl. DIN EN ISO 6186: 1998-08).

Die erfindungsgemäßen, pulverförmigen Zusammensetzungen zeichnen sich dadurch aus, dass der flüssige Kern durch mechanische Belastung der Kern-Hülle-Teilchen, insbesondere durch Reibung und/oder Druck, aus dem Kern-Hülle-Teilchen freigesetzt wird und sich dabei aus der pulverförmigen Zusammensetzung eine Flüssigkeit bildet. Es handelt sich somit um eine pulverförmige powder-to-liquid Zusammensetzung. Die erfindungsgemäßen pulverförmigen Zusammensetzungen können sehr einfach dosiert werden. Sie lassen sich zudem sehr gleichmäßig im Haar verteilen, da der flüssige Kern erst unter mechanischer Beanspruchung am Wirkort freigesetzt und eine gezielte Benetzung der Haarfasern ermöglicht wird. Das Pulver kann also zunächst vorsichtig im Haar verteilt und erst anschließend stärker mechanisch belastet werden, beispielsweise durch gezieltes Einmassieren des Pulvers in das Haar. Dadurch wird der Stylingeffekt erst direkt auf der gewünschten Haarpartie entfaltet.

Die verwendeten pulverförmigen Zusammensetzungen enthalten hydrophobiertes Metalloxid. Die Art des hydrophobierten Metalloxids ist nicht prinzipiell beschränkt, solange sichergestellt ist, dass beim intensiven Vermischen mit der flüssigen, wässrigen Phase ein pulverförmiges Produkt entsteht. Unter hydrophobiert sind im Sinne der Erfindung solche Metalloxide zu verstehen, die zumindest an der Oberfläche der Partikel derart modifiziert wurden, dass das modifizierte Teilchen weniger von Wasser benetzt wird als das nicht modifizierte Teilchen. Insbesondere sind silanisierte, hydrophobierte Metalloxide bevorzugt. Als Reagenz zur Silanisierung des Matalloxids eignet sich erfindungsgemäß bevorzugt mindestens ein Vertreter der Gruppe, die gebildet wird, aus Silanen, Halogensilanen, Alkoxysilanen und Silazanen.

Bevorzugt geeignete hydrophobierte Metalloxide des hydrophobierten Metalloxidpulvers werden erfindungsgemäß ausgewählt aus mindestens einem Vertreter der Gruppe, die gebildet wird aus hydrophobierten Silikaten, hydrophobierten Aluminiumsilikaten, hydrophobiertem Titandioxid sowie hydrophobiertem Siliziumdioxid.

Besonders bevorzugte Aluminiumsilikate (auch Alumosilikate genannt) werden ausgewählt unter Phyllosilikaten, Tectosilikaten oder Gemischen daraus.

Bevorzugt geeignete Phyllosilikate werden ausgewählt unter Kaolinen (hier insbesondere unter Kaolinit, Dickit, Hallosit sowie Nakrit), Serpentin, Talk, Pyrophyllit, Montmorillonit, Quarz, Bentonit, Glimmer (hier insbesondere unter Illit, Muscovit, Paragonit, Phlogopit, Biotit, Lepidolith, Margarit, Smektit (hier insbesondere unter Montmorrilionit, Saponit, Nontronit, Hectorit)).

Bevorzugt geeignete Tectosilikate werden ausgewählt unter Feldspatmineralien (insbesondere Albit, Orthoklas, Anorthit, Leucit, Sodalith, Hauyn, Labradorit, Lasurit, Nosean, Nephelin), Zeolithen.

Die eingesetzten pulverförmigen Zusammensetzungen enthalten das hydrophobierte Metalloxidpulver vorzugsweise in einer Menge von 0,5 bis 30 Gew.-%, bezogen auf das Gewicht der gesamten pulverförmigen Zusammensetzung.

Ferner hat es sich als bevorzugt erwiesen, wenn die hydrophobierten Metalloxide einen Partikeldurchmesser von weniger als 5 µm, bevorzugt weniger als 1 µm, besonders bevorzugt zwischen 20 und 100 nm, aufweisen

Besonders bevorzugt enthält die erfindungsgemäße pulverförmige Zusammensetzung als hydrophobiertes Metalloxidpulver mindestens ein hydrophobiertes Siliziumdioxid, insbesondere mindestens ein silanisiertes, hydrophobiertes Siliziumdioxid.

Als Reagenz zur Silanisierung des Siliziumdioxids eignen sich erfindungsgemäß bevorzugt mindestens ein Vertreter der Gruppe, die gebildet wird, aus Silanen, Halogensilanen, Alkoxysilanen und Silazanen.

Bevorzugte Vertreter der Gruppe der Silane sind Hexa(C₁-C₂₀)alkyldisilane, insbesondere Hexamethyldisilan.

Wenn ein Halogensilan als Silylierungsmittel Anwendung findet, wird als bevorzugtes Halogensilan mindestens eine Verbindung aus der Gruppe ausgewählt, die gebildet wird, aus den Verbindungen

[(C₁-C₂₀)Alkyl]_{z'}SiX_{(4-z')}

X₃Si[(CH₂)n-R]

X₂[(C₁-C₂₀)Alkyl]Si(CH₂)ₙ-R

[(C₁-C₂₀)Alkyl]_{(y'+1)}[R-(CH₂)ₙ]_{(2-y')}SiX

worin
X ein Chlor-, Brom- oder lodatom bedeutet,
z' eine Zahl 1, 2 oder 3 ist,
y' eine Zahl 0, 1 oder 2 ist
n eine ganze Zahl von 1 bis 20 ist und
R steht für einen Rest aus
   (C₁-C₁₀)Alkyl-, Aryl-, (C₁-C₆)Perfluoroalkyl-, -NH₂, -N₃, -SCN, -CH=CH₂, -O(O)C-C(CH₃)=CH₂, -OCH₂-CH=CH₂, 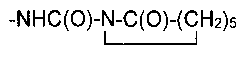 -NH-C(O)O-Me, -NH-C(O)O-Et, -NH-(CH₂)₃-Si(O(C₁-C₆)alkyl)₃.

Wenn ein Alkoxysilan als Silylierungsmittel verwendet wird, wird als bevorzugtes Alkoxysilan mindestens eine Verbindung aus der Gruppe ausgewählt, die gebildet wird, aus den Verbindungen

[(C₁-C₂₀)AlkylO]_{z}Si(C₁-C₂₀)Alkyl_{(4-z)}

[(C₁-C₂₀)AlkylO]_{z}Si[(CH₂)ₙ-R]_{(4-z)}

[(C₁-C₂₀)AlkylO]₂[(C₁-C₂₀)Alkyl]Si(CH₂)ₙ-R

[(C₁-C₂₀)AlkylO][(C₁-C₂₀)Alkyl]₂Si(CH₂)ₙ-R

[(C₁-C₂₀)AlkylO][(C₁-C₂₀)Alkyl]Si[(CH₂)ₙ-R]₂

(C₁-C₂₀Alkyl)₃SiO-C(CH₃)=N-Si(C₁-C₂₀)Alkyl₃,

worin
n eine ganze Zahl von 1 bis 20 ist und
z eine Zahl 1, 2, oder 3 bedeutet
R steht für einen Rest aus
(C₁-C₂₀)Alkyl-, Aryl-, (C₁-C₆)Perfluoroalkyl-, -NH₂, -N₃, -SCN, -CH=CH₂, -O(O)C-C(CH₃)=CH₂, -OCH₂-CH=CH₂, 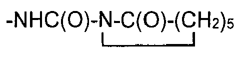
-NH-C(O)O-Me, -NH-C(O)O-Et, -NH-(CH₂)₃-Si(O(C₁-C₆)alkyl)₃.

Als bevorzugtes Silazan wird mindestens eine Verbindung aus der Klasse der Disilazane, insbesondere mindestens eine Verbindung aus Disilazanen der Formel

R'₂R"Si-NH-SiR'₂R"

ausgewählt, worin
R' eine (C₁-C₂₀)Alkylgruppe bedeutet und
R" eine (C₁-C₂₀)Alkylgruppe oder eine Vinylgruppe bedeutet. Ein besonders bevorzugtes Silazan ist Hexamethyldisilazan.

Alle oben genannten Alkylgruppen, ob (C₁-C₆)Alkyl, (C₁-C₁₀)Alkyl oder (C₁-C₂₀)-Alkyl, können sowohl zyklisch, als auch linear bzw. verzweigt sein. Beispiele für erfindungsgemäß verwendbare Alkylgruppen sind Methyl, Ethyl, n-Propyl, Isopropyl, n-Butyl, Cyclopentyl, Cyclohexyl, n-Decyl, Lauryl, Myristyl, Cetyl, Stearyl, Isostearyl und Behenyl.

Ein Beispiel für eine erfindungsgemäße Arylgruppe ist die Phenylgruppe.
Beispiele für eine erfindungsgemäße (C₁-C₆)Perfluoroalkylgruppe sind Trifluormethyl, Perfluoroethyl, Perfluoropropyl und Perfluorohexyl.

Vorzugsweise werden hydrophobierte Siliziumdioxide eingesetzt, die durch Silanisierung von pyrogenem Siliziumdioxid erhalten werden.

Silanisierte, hydrophobierte Siliziumdioxide werden insbesondere bevorzugt ausgewählt aus mindestens einer Verbindung der Gruppe, die gebildet wird aus Trimethylsilylat-beschichtetem Siliziumdioxid, Dimethylsilylat-beschichtetem Siliziumdioxid, Octylsilylat-beschichtetem Siliziumdioxid.

Dabei ist es wiederum bevorzugt das hydrophobierte Metalloxidpulver aus Silica Silylate auszuwählen. Dies sind hydrophobierte Siliziumdioxide, die der INCI-Bezeichnung Silica Silylate gehorchen.

Bevorzugt sind solche hydrophobierte Siliziumdioxide, die eine spezifische Oberfläche nach BET zwischen 10 und 400 m²/g, vorzugsweise zwischen 80 bis 300 m²/g aufweisen. Dabei sind wiederum solche hydrophobierte Siliziumdioide bevorzugt geeignet, die silanisiert sind, insbesondere Silica Silylat.

Eine Vielzahl geeigneter hydrophobierter Siliziumdioxide ist kommerziell erhältlich. Beispielhaft seien Aerosil^{®} R104 V, Aerosil^{®} R106, Aerosil^{®} R202, Aerosil^{®} R805, Aerosil^{®} R812, Aerosil^{®} R812S, Aerosil^{®} R972 und Aerosil^{®} R8200, alle Degussa, sowie HDK^{®} H2000, HDK^{®} H2050 und HDK^{®} H3004, alle Wacker, genannt.

Besonders bevorzugt werden die hydrophobierten Siliziumdioxide eingesetzt, die unter den Bezeichnungen Aerosil^{®} R202, Aerosil^{®} R812S oder Aerosil^{®} R972 erhältlich sind. Ganz besonders bevorzugt kommt das Siliziumdioxid mit der INCI-Bezeichnung Silica Silylate zum Einsatz, das von der Firma Degussa unter der Bezeichnung Aerosil^{®} R812S vertrieben wird.

Die eingesetzten pulverförmigen Zusammensetzungen enthalten das hydrophobierte Siliziumoxidpulver vorzugsweise in einer Menge von 0,5 bis 30 Gew.-%, bezogen auf das Gewicht der gesamten pulverförmigen Zusammensetzung.

Dabei ist es wiederum besonders bevorzugt, das hydrophobierte Siliziumdioxidpulver aus Silica Silylate auszuwählen und in einer Menge von 0,5 bis 30 Gew.-%, bezogen auf das Gewicht der gesamten pulverförmigen Zusammensetzung in der erfindungsgemäßen Zusammensetzung einzusetzen.

Die optimale Menge hängt dabei vor allem von der Hydrophobizität des eingesetzten Siliziumdioxidpulvers ab. Je hydrophober das Siliziumdioxidpulver ist, desto weniger davon wird benötigt, um ein stabiles, pulverförmiges Produkt zu erhalten.

Bevorzugt enthält die erfindungsgemäße pulverförmige Zusammensetzung als hydrophobiertes Metalloxid mindestens ein hydrophobiertes Phyllosilikat, insbesondere mindestens einen hydrophobierten Glimmer und/oder mindestens einen hydrophobierten Talk. Dabei eignet sich wiederum mindestens ein silanisiertes, hydrophobiertes Phyllosilikat bzw. mindestens ein silanisierter hydrophobierter Glimmer und/oder mindestens ein hydrophobierter Talk bevorzugt. Als Reagenz zur Silanisierung des Phyllosilikats bzw. des Glimmers eignen sich erfindungsgemäß bevorzugt mindestens ein Vertreter der Gruppe, die gebildet wird, aus Silanen, Halogensilanen, Alkoxysilanen und Silazanen. Für die besonders bevorzugten Reaganzien wird *mutatis mutandis* auf die Ausführungen zur Silanisierung des Siliziumdioxids verwiesen (vide *supra*).

Silanisierte, hydrophobierte Glimmer werden insbesondere bevorzugt ausgewählt aus mindestens einer Verbindung der Gruppe, die gebildet wird aus Trimethylsilylat-beschichtetem Glimmer, Dimethylsilylat-beschichtetem Glimmer, Octylsilylat-beschichtetem Glimmer.

Silanisierter, hydrophobierter Talk wird insbesondere bevorzugt ausgewählt aus mindestens einer Verbindung der Gruppe, die gebildet wird aus Trimethylsilylat-beschichtetem Talk, Dimethylsilylat-beschichtetem Talk, Octylsilylat-beschichtetem Talk.

Als erfindungsgemäß einsetzbares hydrophob modifiziertes Phyllosilikat sind beispielsweise mit Triethoxycaprylsilan silanisierter, hydrophobierter Glimmer mit Triethoxycaprylsilan silanisierter, hydrophobierter Talk beispielsweise von der Firma LCW zu nennen.

Die eingesetzten pulverförmigen Zusammensetzungen enthalten den hydrophobierten Glimmer und/oder den hydrophobierten Talk vorzugsweise in einer Menge von 0,5 bis 30 Gew.-%, bezogen auf das Gewicht der gesamten pulverförmigen Zusammensetzung.

Die erfindungsgemäßen pulverförmigen Zusammensetzungen enthalten weiterhin zwingend Glycerin.

Der flüssige Kern kann neben dem besagten Glycerin zusätzlich mindestens ein weiteres Lösemittel enthalten. Dies ist insbesondere dann bevorzugt, wenn ausschließlich solche Polyole der Formel (I) eingesetzt werden, die bei 20°C und einem Druck von 1 atm in Form eines Feststoffs vorliegen. Die zusätzlichen Lösemittel sind bevorzugt in einer Menge von 0 bis 40 Gew.-%, besonders bevorzugt von 0 bis 20 Gew.-%, ganz besonders bevorzugt von 0 bis 10 Gew.-%, jeweils bezogen auf das Gewicht des flüssigen Kerns, in der erfindungsgemäßen pulverförmigen Zusammensetzung enthalten.

Das zusätzliche Lösemittel ist selbstredend von dem Glycerin verschieden. Vorzugsweise werden als zusätzliches Lösemittel Wasser oder ein Gemisch aus Wasser und maximal 60 Gew.-% C₁-C₄-Alkohol, bezogen auf das Lösemittelgemisch, eingesetzt. Besonders bevorzugte zusätzliche Lösemittel sind Wasser oder ein Gemisch aus Wasser und maximal 30 Gew.-% C₁-C₄-Alkohol, bezogen auf das Lösemittelgemisch. Ganz besonders bevorzugt wird als zusätzliches Lösemittel Wasser, insbesondere in den oben für das zusätzliche Lösemittel angegebenen bevorzugten Mengen, eingesetzt.

Folgende Ausführungsformen (A) bis (P) eignen sich besonders bevorzugt:
(C): Pulverförmige Zusammensetzungen umfassend Kern-Hülle-Teilchen, deren Hülle Partikel mindestens eines hydrophobierten Metalloxidpulvers enthält und deren flüssiger Kern bezogen auf das Gewicht des Kerns 20,0 Gew.-% bis 100,0 Gew.-%, insbesondere 50,0 Gew.-% bis 100,0 Gew.-%, Glycerin enthält.
(D): Pulverförmige Zusammensetzungen umfassend Kern-Hülle-Teilchen,
   - deren Hülle Partikel mindestens eines hydrophobierten Metalloxidpulvers aus mindestens einem Vertreter der Gruppe, die gebildet wird aus hydrophobierten Silikaten, hydrophobierten Aluminiumsilikaten, hydrophobiertem Titandioxid sowie hydrophobiertem Siliziumdioxid, enthält, und
   - deren flüssiger Kern bezogen auf das Gewicht des Kerns 20,0 Gew.-% bis 100,0 Gew.-%, insbesondere 50,0 Gew.-% bis 100,0 Gew.-%, Glycerin enthält.
(H): Pulverförmige Zusammensetzungen umfassend Kern-Hülle-Teilchen,
   - deren Hülle Partikel mindestens eines hydrophobierten Metalloxidpulvers aus mindestens einem Vertreter der Gruppe, die gebildet wird aus hydrophobierten Silikaten, hydrophobierten Aluminiumsilikaten, hydrophobiertem Titandioxid sowie hydrophobiertem Siliziumdioxid, enthält, und
   - deren flüssiger Kern bezogen auf das Gewicht des Kerns
      (i) 20,0 Gew.-% bis 100,0 Gew.-%, insbesondere 50,0 Gew.-% bis 100,0 Gew.-%, Glycerin und
      (ii) 0 bis 60 Gew.-%, insbesondere 0 bis 10 Gew.-%, Wasser enthält.
(K): Pulverförmige Zusammensetzungen umfassend Kern-Hülle-Teilchen, deren Hülle bezogen auf das Gewicht der pulverförmigen Zusammensetzung 0,5 bis 30,0 Gew.-% Partikel mindestens eines hydrophobierten Metalloxidpulvers enthält und deren flüssiger Kern bezogen auf das Gewicht des Kerns 20,0 Gew.-% bis 100,0 Gew.-%, insbesondere 50,0 Gew.-% bis 100,0 Gew.-%, Glycerin enthält.
(L): Pulverförmige Zusammensetzungen umfassend Kern-Hülle-Teilchen,
   - deren Hülle bezogen auf das Gewicht der pulverförmigen Zusammensetzung 0,5 bis 30,0 Gew.-% Partikel mindestens eines hydrophobierten Metalloxidpulvers aus mindestens einem Vertreter der Gruppe, die gebildet wird aus hydrophobierten Silikaten, hydrophobierten Aluminiumsilikaten, hydrophobiertem Titandioxid sowie hydrophobiertem Siliziumdioxid, enthält, und
   - deren flüssiger Kern bezogen auf das Gewicht des Kerns 20,0 Gew.-% bis 100,0 Gew.-%, insbesondere 50,0 Gew.-% bis 100,0 Gew.-%, Glycerin enthält.
(O): Pulverförmige Zusammensetzungen umfassend Kern-Hülle-Teilchen, deren Hülle bezogen auf das Gewicht der pulverförmigen Zusammensetzung 0,5 bis 30,0 Gew.-% Partikel mindestens eines hydrophobierten Metalloxidpulvers enthält und deren flüssiger Kern bezogen auf das Gewicht des Kerns
   (i) 20,0 Gew.-% bis 100,0 Gew.-%, insbesondere 50,0 Gew.-% bis 100,0 Gew.-%, Glycerin und
   (ii) 0 bis 60 Gew.-%, insbesondere 0 bis 10 Gew.-%, Wasser enthält.
(P): Pulverförmige Zusammensetzungen umfassend Kern-Hülle-Teilchen,
   - deren Hülle bezogen auf das Gewicht der pulverförmigen Zusammensetzung 0,5 bis 30,0 Gew.-% Partikel mindestens eines hydrophobierten Metalloxidpulvers aus mindestens einem Vertreter der Gruppe, die gebildet wird aus hydrophobierten Silikaten, hydrophobierten Aluminiumsilikaten, hydrophobiertem Titandioxid sowie hydrophobiertem Siliziumdioxid, enthält, und
   - deren flüssiger Kern bezogen auf das Gewicht des Kerns
      (i) 20,0 Gew.-% bis 100,0 Gew.-%, insbesondere 50,0 Gew.-% bis 100,0 Gew.-%, Glycerin und
      (ii) 0 bis 60 Gew.-%, insbesondere 0 bis 10 Gew.-%, Wasser enthält.

Für die Ausführungsformen (C) bis (P) gelten wiederum *mutatis mutandis* die genannten bevorzugten Ausführungsformen der Inhaltsstoffe (*vide supra*) als bevorzugt. Insbesondere wird im Rahmen der bevorzugten Ausführungsformen (C) bis (P) als bevorzugtes hydrophobiertes Metalloxidpulver mindestens ein hydrophobiertes Siliziumdioxid, insbesondere mindestens ein silanisiertes, hydrophobiertes Siliziumdioxid, eingesetzt.

Die flüssige, wässrige Phase kann zusätzlich bevorzugt solche Zusatzstoffe enthalten, die die Oberflächenspannung der wässrigen Phase nicht signifikant herabsetzen. Daher ist es bevorzugt, wenn die erfindungsgemäßen pulverförmigen Zusammensetzungen im flüssigen Kern nicht mehr als 0,01 Gew.-% Tenside bezogen auf das Gewicht der pulverförmigen Zusammensetzung enthalten.

Zur Verbesserung des Griffs kann die erfindungsgemäße pulverförmige Zusammensetzung zusätzlich mindestens ein Mineralsalz enthalten. Bevorzugte Mineralsalze werden ausgewählt aus hygroskopischen Mineralsalzen wie insbesondere Calciumchlorid, Magnesiumchlorid, Magnesiumsulfat und Natriumsulfat. Die Mineralsalze sind wiederum bezogen auf das Gewicht der pulverförmigen Zusammensetzung bevorzugt in einer Menge von 1,0 bis 30,0 Gew.-%, insbesondere von 4,0 bis 25 Gew.-%, enthalten.

Zur Verbesserung der Haarpflege können die erfindungsgemäßen pulverförmigen Zusammensetzungen bevorzugt mindestens einen Pflegestoff enthalten, der sich bevorzugt in der flüssigen, wässrigen Phase befindet.

Die erfindungsgemäßen pulverförmigen Zusammensetzungen können als Pflegestoff mindestens einen UV-Filter. Die erfindungsgemäß geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.
Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, dass bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyldimethylaminobenzamidopropyl-dimethylammoniumtosylat (Escalol^{®} HP 610).
Selbstverständlich umfasst die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter sind in den erfindungsgemäßen pulverförmigen Zusammensetzungen üblicherweise in Mengen 0,01-5 Gew.-%, bezogen auf die erfindungsgemäße pulverförmige Zusammensetzung, enthalten. Mengen von 0,1-2,5 Gew.-% sind bevorzugt.

Die erfindungsgemäßen Zubereitungen enthalten als zusätzlichen Pflegestoff bevorzugt mindestens ein Vitamin, ein Provitamin, eine Vitaminvorstufe sowie eines derer Derivate.
Dabei sind erfindungsgemäß solche Vitamine, Pro-Vitamine und Vitaminvorstufen bevorzugt, die üblicherweise den Gruppen A, B, C, E, F und H zugeordnet werden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist ein erfindungsgemäß ganz besonders bevorzugtes Vitamin. Durch Einsatz von Biotin in den erfindungsgemäßen Zubereitungen konnte überraschender Weise die Restrukturierung der Fasern verbessert werden, eine Strukturstabilisierung erzielt werden sowie das Reizpotential der Mittel wesentlich reduziert werden. Biotin ist in den erfindungsgemäßen pulverförmigen Zusammensetzungen bevorzugt in Mengen von 0,0001 bis 2,0 Gew.-%, insbesondere in Mengen von 0,001 bis 0,2 Gew.-%, jeweils bezogen auf die erfindungsgemäße pulverförmige Zusammensetzung enthalten.

Bevorzugt enthalten die erfindungsgemäßen pulverförmigen Zusammensetzungen Vitamine, Provitamine und Vitaminvorstufen aus den Gruppen A, B, C, E und H. Das Vitamin C kann erfindungsgemäß ganz besonders bevorzugt sein.

Panthenol, Pantolacton, Pyridoxin, Derivate des Pyridoxins, Nicotinsäureamid, Biotin und Mischungen davon sind erfindungsgemäß besonders bevorzugte Pflegestoffe.

Die erfindungsgemäßen pulverförmigen Zusammensetzungen können als Pflegestoff mindestens einen Pflanzenextrakt enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Besonders bevorzugt sind die Extrakte aus Moringa Olifeira, Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel. Ganz besonders geeignet sind die Extrakte aus Moringa Olifeira, Grünem Tee, Mandel, Aloe Vera, Kokosnuss, Mango, Aprikose, Limone, Weizen, Kiwi und Melone. Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen. Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Zubereitungen Mischungen aus mehreren, insbesondere aus zwei, verschiedenen Pflanzenextrakten einzusetzen.
Die erfindungsgemäßen pulverförmigen Zusammensetzungen können als Pflegestoff mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton. Erfindungsgemäß können Proteinhydrolysate sowohl pflanzlichen als auch tierischen oder marinen oder synthetischen Ursprungs eingesetzt werden.
Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben. Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Im Rahmen einer neunten bevorzugten Ausführungsform enthalten die erfindungsgemäßen Zubereitungen als Pflegestoff Ectoin oder Ectoinderivate, Allantoin, Taurin und Bisabolol enthalten.

Unter dem Begriff Aminosäure werden die stereoisomeren Formen, z.B. D- und L-Formen, folgender Verbindungen verstanden: Asparagin, Arginin, Asparaginsäure, Glutamin, Glutaminsäure, β-Alanin, γ-Aminobutyrat, N_{ε}-Acetyllysin, N_{δ}-Acetylornitin, N_{γ}-Acetyldiaminobutyrat, N_{α}-Acetyldiaminobutyrat, Histidin, Isoleucin, Leucin, Methionin, Phenylalanin, Serin, Threonin und Tyrosin.

L-Aminosäuren sind bevorzugt. Aminosäurereste leiten sich von den entsprechenden Aminosäuren ab. Die folgenden Aminosäurereste sind bevorzugt:
Gly, Ala, Ser, Thr, Val, β-Ala, γ-Aminobutyrat, Asp, Glu, Asn, Aln, N_{ε}-Acetyllysin, N_{δ}-Acetylornithin, N_{γ}-Acetyidiaminobutyrat, N_{α}-Acetyldiaminobutyrat.

Die Kurzschreibweise der Aminosäuren erfolgte nach der allgemein üblichen Schreibweise. Die Di- oder Tripeptidreste sind in ihrer chemischen Natur nach Säureamide und zerfallen bei der Hydrolyse in 2 oder 3 Aminosäuren. Die Aminosäuren in dem Di- oder Tripeptidrest sind durch Amidbindungen miteinander verbunden.

Die erfindungsgemäßen pulverförmigen Zusammensetzungen enthalten diese Wirkstoffe bevorzugt in Mengen von 0,001 bis 2, insbesondere von 0,01 bis 0,5 Gew.-%, jeweils bezogen auf die erfindungsgemäße pulverförmige Zusammensetzung.

Alle vorgenannten bevorzugten optionalen Pflegestoffe sind bevorzugt Bestandteil des flüssigen Kerns der erfindungsgemäßen pulverförmigen Zusammensetzungen.

Die erfindungsgemäßen pulverförmigen Zusammensetzungen enthalten zusätzlich mindestens ein kationisches, amphoteres oder anionisches festigendes Polymer. Dabei können die festigenden Polymere im flüssigen Kern dispergiert oder gelöst vorliegen, und/oder die festigenden Polymere sind als partikelförmiger Feststoff Bestandteil der Hülle der Kern-Hülle-Teilchen.

Unter Polymeren werden erfindungsgemäß Verbindungen verstanden, die aus einer Vielzahl von Molekülen aufgebaut sind, in denen eine Art oder mehrere Arten von Atomen oder Atomgruppierungen (sogenannte konstitutive Einheiten, Grundbausteine oder Wiederholungseinheiten) wiederholt aneinander gereiht sind und die ein Molekulargewicht von wenigstens 10000 g/mol besitzen. Die Polymere werden durch Polyreaktion erhalten, wobei letztere künstlich (d.h. synthetisch) oder natürlich erfolgen kann.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser, Alkohol oder Wasser/Alkohol-Gemischen besitzen. So lassen sich entsprechende Lösungen herstellen, die sich auf einfache Art und Weise anwenden bzw. weiterverarbeiten lassen.

Unter filmbildenden Polymeren werden weiterhin solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Die zusätzlichen festigenden Polymere sind bevorzugt in einer Menge von 0,1 Gew.-% bis 10 Gew.-%, insbesondere von 1,0 Gew.-% bis 8,0 Gew.-%, ganz besonders bevorzugt von 2,0 bis 6,0 Gew.-%, jeweils bezogen auf das Gewicht der pulverförmigen Zusammensetzung, enthalten. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert der Zusammensetzung eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Ein erfindungsgemäß bevorzugt geeignetes kationisches festigendes Polymer ist mindestens ein kationisches festigendes Polymer, das mindestens ein Strukturelement der Formel (M9) und zusätzlich mindestens ein Strukturelement der Formel (M10) enthält 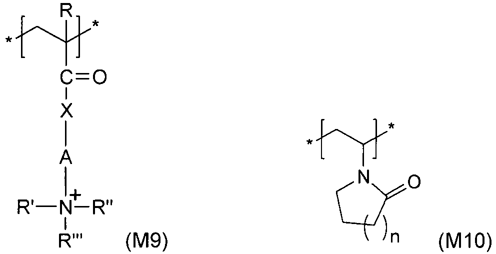 worin
- R: für ein Wasserstoffatom oder eine Methylgruppe steht,
- R', R" und R": unabhängig voneinander stehen für eine (C₁ bis C₃₀)-Alkylgruppe,
- X: steht für ein Sauerstoffatom oder eine Gruppe NH,
- A: steht für eine Ethan-1,2,-diylgruppe oder eine Propan-1,3-diylgruppe,
- n: 1 oder 3 bedeutet.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Solche Verbindungen sind beispielsweise als
- Copolymere aus mit Diethylsulfat quaterniertem Dimethylaminoethylmethacrylat, mit Vinylpyrrolidon mit der INCl-Bezeichnung Polyquaternium-11 unter den Bezeichnungen Gafquat^{®} 440, Gafquat^{®}734, Gafquat^{®}755 (jeweils Firma ISP) sowie Luviquat PQ 11 PN (Firma BASF SE),
- Copolymere aus N-Vinylpyrrolidon, N-(3-Dimethylaminopropyl)methacrylamid und 3-(Methacryloylamino)propyl-lauryl-dimethylammoniumchlorid (INCI-Bezeichnung: Polyquaternium-55), welches beispielsweise unter dem Handelsnamen Styleze W 10 oder W 20 (10 bzw. 20 Gew.-% Aktivsubstanz in Ethanol-Wasser-Gemisch) von der Firma ISP vertrieben wird.
- Copolymere aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle^{®} 300 (28-32 Gew.-% Aktivsubstanz in Ethanol-Wasser-Gemisch) von der Firma ISP vertrieben wird.

Als im Sinne der Erfindung besonders bevorzugt verwendbare kationische Polymere dienen weiterhin solche kationischen festigenden Copolymere, die mindestens ein Strukturelement der Formel (M11) 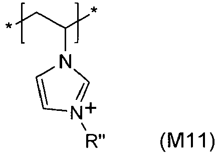 worin R" für eine (C₁ bis C₄)-Alkylgruppe, insbesondere eine Methylgruppe, steht,
und zusätzlich mindestens ein weiteres kationisches und/oder nichtionisches Strukturelement aufweisen.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Es ist wiederum erfindungsgemäß bevorzugt, wenn als zusätzliches kationisches festigendes Polymer, mindestens ein Copolymer (c1) enthalten ist, das neben mindestens einem Strukturelement der Formel (M11) zusätzlich ein Strukturelement der Formel (M6) umfasst 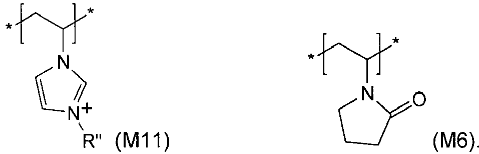 worin R" für eine (C₁ bis C₄)-Alkylgruppe, insbesondere eine Methylgruppe, steht.

Zur Kompensation der positiven Polymerladung der Copolymere (c1) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Ganz besonders bevorzugte kationische festigende Polymere als Copolymere (c1) enthalten 10 bis 30 Mol-%, vorzugsweise 15 bis 25 Mol.-% und insbesondere 20 Mol.-% Struktureinheiten gemäß Formel (M11) und 70 bis 90 Mol.-%, vorzugsweise 75 bis 85 Mol.-% und insbesondere 80 Mol.-% Struktureinheiten gemäß Formel (M6).

Hierbei ist besonders bevorzugt, wenn die Copolymere (c1) neben Polymereinheiten, die aus dem Einbau der genannten Struktureinheiten gemäß Formel (M11) und (M6) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c1) ausschließlich aus Struktureinheiten der Formel (M11) mit R" = Methyl und (M6) aufgebaut.

Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat^{®} Style, Luviquat^{®} FC 370, Luviquat^{®} FC 550, Luviquat^{®} FC 905 und Luviquat^{®} HM 552

Wird in zur Kompensation der positiven Ladung des Polymers der Formel (Poly1) ein Methosulfat verwendet, so werden diese N-Methylvinylimidazol/Vinylpyrrolidon-Copolymere werden laut INCl-Nomenklatur als Polyquaternium-44 bezeichnet und sind beispielsweise von der BASF unter den Handelsnamen Luviquat^{®} UltraCare erhältlich.

Zusätzlich zu dem bzw. den Copolymer(en) (c1) oder an dessen bzw. deren Stelle können die erfindungsgemäßen pulverförmigen Zusammensetzungen auch Copolymere (c2) enthalten, die ausgehend vom Copolymer (c1) als zusätzliche Struktureinheiten Struktureinheiten der Formel (M7) aufweisen

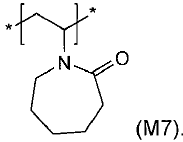

Weitere besonders bevorzugte erfindungsgemäße pulverförmige Zusammensetzungen sind somit dadurch gekennzeichnet, daß sie als kationisches festigendes Polymer mindestens ein Copolymer (c2) enthalten, das mindestens eine Struktureinheit gemäß Formel (M11-a) und mindestens eine Struktureinheit gemäß Formel (M6) und mindestens eine Struktureinheit gemäß Formel (M7) enthält

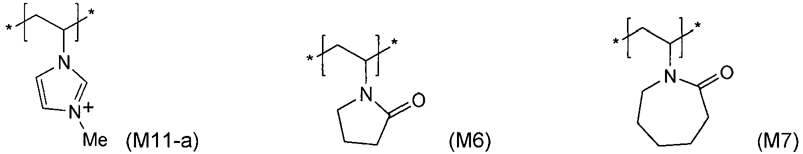

Auch hierbei ist besonders bevorzugt, wenn die Copolymere (c2) neben Polymereinheiten, die aus dem Einbau der genannten Struktureinheiten gemäß Formel (M11-a), (M6) und (M7) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c2) ausschließlich aus Struktureinheiten der Formeln (M11-a), (M6) und (M7) aufgebaut.

Zur Kompensation der positiven Polymerladung der Komponente (c2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat^{®} Hold erhältlich.

Ganz besonders bevorzugte Copolymere (c2) enthalten 1 bis 20 Mol-%, vorzugsweise 5 bis 15 Mol.-% und insbesondere 10 Mol.-% Struktureinheiten gemäß Formel (M11-a) und 30 bis 50 Mol.-%, vorzugsweise 35 bis 45 Mol.-% und insbesondere 40 Mol.-% Struktureinheiten gemäß Formel (M6) und 40 bis 60 Mol.-%, vorzugsweise 45 bis 55 Mol.-% und insbesondere 60 Mol.-% Struktureinheiten gemäß Formel (M7).

Zusätzlich zu dem bzw. den Copolymer(en) (c1) und/oder (c2) oder an dessen bzw. deren Stelle können die erfindungsgemäßen pulverförmigen Zusammensetzungen als festigendes kationisches Polymer auch Copolymere (c3) enthalten, die als Struktureinheiten Struktureinheiten der Formeln (M11-a) und (M6) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße pulverförmige Zusammensetzungen sind dadurch gekennzeichnet, daß sie als zusätzliches kationisches festigendes Polymer mindestens ein Copolymer (c3) enthalten, das mindestens eine Struktureinheit gemäß Formel (M11-a) und mindestens eine Struktureinheit gemäß Formel (M6) und mindestens eine Struktureinheit gemäß Formel (M10) und mindestens eine Struktureinheit gemäß Formel (M12) enthält

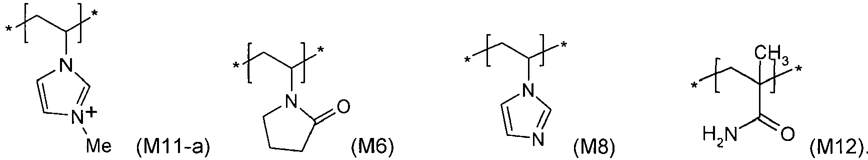

Auch hierbei ist besonders bevorzugt, wenn die Copolymere (c3) neben Polymereinheiten, die aus dem Einbau der genannten Struktureinheiten gemäß Formel (M11-a), (M6), (M8) und (M12) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c3) ausschließlich aus Struktureinheiten der Formel (M11-a), (M6), (M8) und (M12) aufgebaut.

Zur Kompensation der positiven Polymerladung der Komponente (c3) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat^{®} Supreme erhältlich.

Ganz besonders bevorzugte Copolymere (c3) enthalten 1 bis 12 Mol-%, vorzugsweise 3 bis 9 Mol.-% und insbesondere 6 Mol.-% Struktureinheiten gemäß Formel (M11-a) und 45 bis 65 Mol.-%, vorzugsweise 50 bis 60 Mol.-% und insbesondere 55 Mol.-% Struktureinheiten gemäß Formel (M6) und 1 bis 20 Mol.-%, vorzugsweise 5 bis 15 Mol.-% und insbesondere 10 Mol.-% Struktureinheiten gemäß Formel (M8) und 20 bis 40 Mol.-%, vorzugsweise 25 bis 35 Mol.-% und insbesondere 29 Mol.-% Struktureinheiten gemäß Formel (M12).

Unter den zusätzlichen festigenden Polymeren ausgewählt aus den kationischen Polymeren mit mindestens einem Strukturelement der obigen Formel (M11-a), gelten als bevorzugt:
- Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat^{®} Style, Luviquat^{®} FC 370, Luviquat^{®} FC 550, Luviquat^{®} FC 905 und Luviquat^{®} HM 552 (BASF SE)),
- Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat^{®} Care (BASF SE)),
- Vinylpyrrolidon/Vinylcaprolactam/1-Vinyl-3-methyl-1H-imidazolium-Terpolymer (wie beispielsweise das mit der INCI-Bezeichnung Polyquaternium-46 unter den Handelsbezeichnungen Luviquat^{®} Care oder Luviquat^{®} Hold (BASF SE)),
- Vinylpyrrolidon/Methacrylamid/Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat^{®} Supreme (BASF SE)),
sowie Gemische aus diesen Polymeren.

Weitere in den erfindungsgemäßen pulverförmigen Zusammensetzungen bevorzugt einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Als im Sinne der Erfindung bevorzugt geeignete temporär kationische Polymere gelten gleichfalls solche, die mindestens eine Struktureinheit der Formeln (M1-1) bis (M1-8) aufweisen

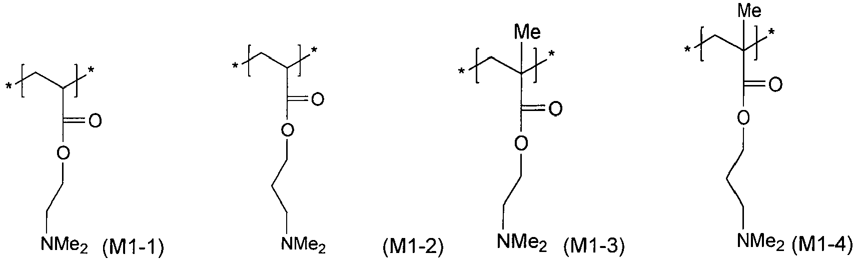

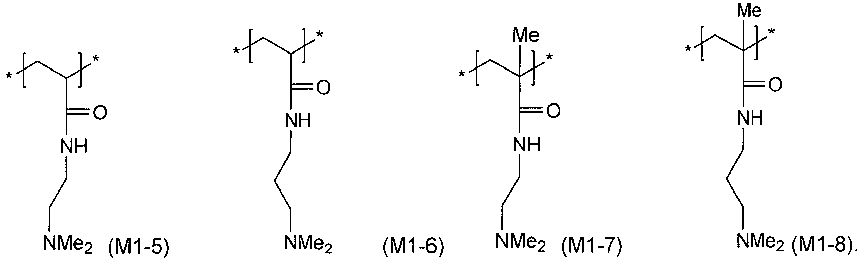

Dabei sind wiederum solche Copolymere bevorzugt, die mindestens eine Struktureinheit der Formeln (M1-1) bis (M1-8) und zusätzlich mindestens eine Struktureinheit der Formel (M10) enthalten, 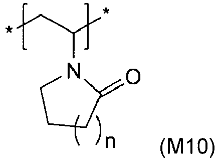 worin
- n: 1 oder 3 bedeutet.
Dabei gilt wiederum die Gruppe der Polymere
- Vinylcaprolactam/Vinylpyrrolidon/Dimethylaminoethylmethacrylat-Copolymer (beispielsweise INCI-Bezeichnung: Vinyl Caprolactam/PVP/Di-methylaminoethyl Methacrylate Copolymer unter dem Handelsnamen Gaffix^{®} VC 713 (ISP)),
- Vinylpyrrolidon/Vinylcaprolactam/Dimethylaminopropylmethacrylamid-Copolymer (beispielsweise INCI-Bezeichnung: VP/Vinyl Caprolactam/DMAPA Acrylates Copolymer unter dem Handelsnamen Aquaflex^{®} SF-40 (ISP)),
- Vinylcaprolactam/Vinylpyrrolidon/Dimethylaminoethylmethacrylat-Copolymer (beispielsweise als.35-39% Festkörper in Ethanol in form des Handelsprodukts Advantage LC E mit der INCI-Bezeichnung: Vinyl Caprolactam/VP/Dimethylaminoethyl Methacrylate Copolymer, Alcohol, Lauryl Pyrrolidone (ISP)),
- Vinylpyrrolidon/Dimethylaminopropylmethacrylamid-Copolymer (beispielsweise INCl-Bezeichnung: VP/DMAPA Acrylates Copolymer unter dem Handelsnamen Styleze CC-10 (ISP)),
als bevorzugte Liste zur Auswahl mindestens eines oder mehrerer Polymere daraus.

Die erfindungsgemäßen pulverförmigen Zusammensetzungen können als festigendes Polymer auch mindestens ein amphoteres festigendes Polymer enthalten. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder - SO₃⁻-Gruppen enthalten, und solche Polymere zusammengefaßt, die -COOH- oder SO₃H-Gruppen und quartäre Ammoniumgruppen enthalten.
Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Alkylestern darstellt.
Die amphoteren festigenden Polymere sind in den erfindungsgemäßen pulverförmigen Zusammensetzungen bevorzugt in Mengen von 0,01 Gew.-% bis 20 Gew.-%, besonders bevorzugt von 0,05 bis 10 Gew.-%, bezogen auf die gesamte Zusammensetzung, enthalten. Mengen von 0,1 bis 5,0 Gew.-% sind ganz besonders bevorzugt.

Weiterhin können als festigende Polymere mindestens ein anionisches festigendes Polymer eingesetzt werden. Bei den anionischen Polymeren handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.
Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Weitere bevorzugt einsetzbare anionische Polymere werden ausgewählt aus der Gruppe, die gebildet wird, aus
- Copolymeren aus Vinylacetat und Crotonsäure (wie sie beispielsweise als Handelsprodukt Aristoflex^{®} A 60 mit der INCI-Bezeichnung VA/Crotonates Copolymer von der Firma CIBA in einer 60 Gew.-%-igen Dispersion in Isopropanol-Wasser vermarktet werden),
- Copolymeren aus Ethylacrylat und Methacrylsäure (wie sie beispielsweise unter dem Handelsnamen Luviflex^{®} Soft mit einer Säurezahl von 84 bis 105 unter der INCI-Bezeichnung Acrylates Copolymer in einer ca. 20 bis 30 Gew.-%igen Dispersion in Wasser von der Firma BASF SE vertrieben werden),
- Polyurethanen mit mindestens einer Carboxylgruppe (wie beispielsweise ein Copolymer aus Isophthalsäure, Adipinsäure, 1,6-Hexandiol, Neopentylglykol und Isophorondiisocyanat wie es unter dem Handelsnamen Luviset PUR mit der INCI-Bezeichnung Polyurethane-1 von der Firma BASF SE vertrieben wird).

Die erfindungsgemäßen pulverförmigen Zusammensetzungen können in nahezu beliebigen Behältnissen konfektioniert werden. Es muss lediglich sichergestellt werden, dass die mechanische Belastung des Pulvers bei der Entnahme der Zusammensetzung nicht so hoch ist, dass bereits bei der Entnahme das Pulver in flüssige Form überführt wird. Geeignet sind beispielsweise Tiegel, Flaschen oder auch Tetrapacks, wobei das Behältnis beispielsweise mit einer Schütt- und Dosiervorrichtung ausgestaltet werden kann.

Alle erfindungsgemäßen pulverförmigen Zusammensetzungen können zumindest durch folgendes bevorzugtes Herstellungsverfahren bereitgestellt werden, welches der zweite Gegenstand der Erfindung ist:
Ein zweiter Gegenstand der Erfindung ist die Verwendung einer pulverförmigen Zusammensetzung des ersten Erfindungsgegenstandes zur temporären Verformung keratinhaltiger Fasern, insbesondere menschlicher Haare.

Ein dritter Erfindungsgegenstand ist ein Verfahren zur temporären Verformung keratinhaltiger Fasern, insbesondere menschlicher Haare, in dem
- eine pulverförmige Zusammensetzung gemäß erstem Erfindungsgegenstand vor, während oder nach dem Auftragen auf die keratinhaltigen Fasern einer mechanischen Belastung ausgesetzt wird, wobei sich die pulverförmige Zusammensetzung in eine Flüssigkeit umwandelt,
- die keratinhaltigen Fasern
   - vor, nach oder während des Auftragens der pulverförmigen Zusammensetzung und/oder
   - vor, nach oder während der mechanischen Belastung der pulverförmigen Zusammensetzung
   in Form gebracht werden,
   - die Form der keratinhaltigen Fasern durch die in besagte Flüssigkeit umgewandelte pulverförmige Zusammensetzung temporär fixiert wird.

Bei der Verwendung der pulverförmigen Zusammensetzung zur temporären Verformung keratinischer Fasern wird zunächst bevorzugt die gewünschte Menge der pulverförmigen Zusammensetzung dem Behältnis entnommen. Die Zusammensetzung kann dabei direkt auf die zu behandelnde keratinische Faser oder aber beispielsweise auf die Hand gegeben werden. Im ersten Fall kann das aufgebrachte Pulver direkt auf der keratinischen Faser einer mechanischen Belastung, beispielsweise mittels der Hände, ausgesetzt werden, wodurch die flüssige, wässrige Phase direkt auf der Faser freigesetzt und die Wirkung des in Form von Partikeln vorliegenden filmbildenden und/oder festigenden Polymer entfaltet werden. Wird die pulverförmige Zusammensetzung zunächst auf die Hand gegeben, so kann es zunächst vorsichtig im Haar verteilt und wiederum erst anschließend stärker mechanisch belastet werden, beispielsweise durch gezieltes Einmassieren des Pulvers in das Haar. Dadurch wird die flüssige, wässrige Phase direkt auf der Faser freigesetzt und die Wirkung des in Form von Partikeln vorliegenden filmbildenden und/oder festigenden Polymer entfaltet. So lässt sich sehr gezielt eine hervorragende Stylingwirkung erreichen. Es ist natürlich auch möglich, die pulverförmige Zusammensetzung bereits auf der Hand zu verreiben und erst das entstehende flüssige oder pastenartige Mittel auf die keratinische Faser aufzubringen. Diese Vorgehensweise ist allerdings nicht bevorzugt, da dabei auf einen wesentlichen Vorteil der pulverförmigen Konsistenz des Stylingmittels, nämlich die gute Verteilbarkeit, verzichtet wird. Die pulverförmige Zusammensetzung lässt sich natürlich auch mit einem Hilfsmittel, etwa einem Pinsel, einem Schwamm, einem Tuch, einer Bürste oder einem Kamm auftragen.

Die nachfolgenden Beispiele sollen den Gegenstand der vorliegenden Erfindung erläutern ohne ihn in irgendeiner Weise zu beschränken.

### Beispiele

Die im Folgenden angegebenen Mengenangaben verstehen sich - soweit nichts anderes vermerkt ist - in Gewichtsprozent.

### 1. Herstellung pulverförmiger Stylingmittel

Es wurden wie nachfolgend beschrieben die pulverförmigen Stylingmittel V1, E1 bis E6 hergestellt, wobei diese folgende Zusammensetzung aufwiesen:

| **Rohstoffe** | **V1** | **E1** | **E2** | **E3** | **E4** | **E5** | **E6** |
|---|---|---|---|---|---|---|---|
| Aerosil^{®} R 812 S | 17,00 | 14,00 | 15,00 | 15,00 | 14,00 | 15,00 | 15,00 |
| Natriumbenzoat | 0,20 | - | 0,40 | - | - | - | - |
| Zitronensäure Monohydrat | 0,02 | - | 0,02 | | - | - | - |
| Amphomer^{®} | 0,05 | - | 0,05 | - | - | - | - |
| Wasser, entsalzt | 82,73 | 8,75 | 0,10 | 0,13 | 0,40 | 0,43 | 25,50 |
| Glycerin | - | 69,65 | 19,90 | 64,87 | 80,00 | 84,57 | - |
| Sorbitol | - | - | - | - | - | - | 59,50 |
| Calciumchlorid-6 H₂O | - | 5,00 | 15,00 | 19,40 | 5,00 | - | - |
| Meersalz, sprühgetrocknet | - | 2,00 | - | - | - | - | - |
| 2-Phenoxyethanol | - | 0,60 | - | 0,60 | 0,60 | - | - |

Die Zusammensetzungen V1, E1 und D3 bis D6 sind nicht erfindungsgemäß. Die Zusammensetzung E2 ist erfindungsgemäß.

Alle Bestandteile bis auf Aerosil^{®} R 812 S und falls vorhanden Amphomer^{®} wurden in einem Gefäß gemischt. Diese Flüssigkeit (Kern) wurde mit dem hydrophobierten Siliziumdioxidpulver Aerosil^{®} R 812 S (INCl-Bezeichnung: Silica Silylate) versetzt. Nach einer Rührzeit von jeweils 30 bis 45 Sekunden hatte sich jeweils ein stabiles Pulver gebildet. Das Amphomer (sofern enthalten) wurde als Feststoff zu dem stabilen Pulver gegeben und unter rühren untergemengt. Das so erhaltene fertige Stylingpulver wurde in Polyethylenflaschen abgefüllt.

### 2. Anwendung

Im Rahmen eines Halbseitentests an einem Proband mit den oben genannten Stylingmitteln V1 und E2 frisiert. Dabei wurde das Kopfhaar halbseitig in eine rechte und eine linke Hälfte eingeteilt. Die linke Hälfte wurde mit dem Vergleichsstylingpulver frisiert, die rechte Hälfte wurde mit einem erfindungsgemäßen Stylingpulver frisiert. Zu diesem Zweck wurden gleiche Mengen des jeweiligen Pulvers im Haar verteilt Anschließend wurde die Zusammensetzung durch massieren und kneten verflüssigt und dabei das Haar mit den Händen in die gewünschte Form gebracht.

Für die erfindungsgemäße Zusammensetzung E2 wurde im Vergleich zur Zusammensetzung V1 Haar mit einem natürlichen Glanz und einem vollen, natürlichen Griff erhalten. Die Fixierung der Frisur war in beiden Fällen vergleichbar. Die mit V1 behandelten Haare wirkten stumpfer, weniger lebendig und klebten leicht.

### 3. Verzeichnis der eingesetzten Rohstoffe

Die im Rahmen der Beispiele eingesetzten Rohstoffe sind wie folgt definiert:

| | |
|---|---|
| Aerosil^{®} R 812 S | INCI-Bezeichnung: Silica Silylate (Evonik Degussa) |
| Amphomer^{®} | amphoteres Polymer mit der INCI-Bezeichnung: Octylacrylamide/Acrylates/Butyl-aminoethyl Methacrylate Copolymer, weißes Pulver (National Starch) |

## Patentansprüche

1. Pulverförmige Zusammensetzung umfassend Kern-Hülle-Teilchen, deren Hülle Partikel mindestens eines hydrophobierten Metalloxidpulvers enthält und deren flüssiger Kern bezogen auf das Gewicht des Kerns 20,0 Gew.-% bis 100,0 Gew.-%, insbesondere 50,0 Gew.-% bis 100,0 Gew.-%, mindestens Glycerid enthält, **dadurch gekennzeichnet, dass** zusätzlich mindestens ein kationisches, amphoteres oder anionisches festigendes Polymer enthalten ist.

2. Pulverförmige Zusammensetzung nach Anspruch 1, **dadurch gekennzeichnet, dass** das hydrophobierte Metalloxid des hydrophobierten Metalloxidpulvers ausgewählt wird aus mindestens einem Vertreter der Gruppe, die gebildet wird aus hydrophobierten Silikaten, hydrophobierten Aluminiumsilikaten, hydrophobiertem Titandioxid sowie hydrophobiertem Siliziumdioxid.

3. Pulverförmige Zusammensetzung nach einem der Ansprüche 1 oder 2, **dadurch gekennzeichnet, dass** hydrophobiertes Metalloxidpulver in einer Menge von 0,5 Gew.-% bis 30 Gew.-% bezogen auf das Gewicht der gesamten pulverförmigen Zusammensetzung enthalten ist.

4. Pulverförmige Zusammensetzung nach einem der Ansprüche 1 bis 3, **dadurch gekennzeichnet, dass** das hydrophobierte Metalloxidpulver ausgewählt wird aus mindestens einem Vertreter der Gruppe, die gebildet wird aus hydrophobierten Silikaten, hydrophobierten Aluminiumsilikaten, hydrophobiertem Titandioxid sowie hydrophobiertem Siliziumdioxid, bevorzugt aus Silica Silylate.

5. Pulverförmige Zusammensetzung nach einem der Ansprüche 1 bis 4, **dadurch gekennzeichnet, dass** zusätzlich mindestens ein weiteres Lösemittel, insbesondere Wasser, enthalten ist, bevorzugt in einer Menge von 0 bis 40 Gew.-%, besonders bevorzugt von 0 bis 20 Gew.-%, ganz besonders bevorzugt von 0 bis 10 Gew.-%, jeweils bezogen auf das Gewicht des flüssigen Kerns, in der erfindungsgemäßen pulverförmigen Zusammensetzung.

6. Pulverförmige Zusammensetzung nach einem der Ansprüche 1 bis 5, **dadurch gekennzeichnet, dass** zusätzlich mindestens ein Mineralsalz enthalten ist.

7. Verwendung einer pulverförmigen Zusammensetzung gemäß einem der Ansprüche 1 bis 6 zur temporären Verformung keratinhaltiger Fasern, insbesondere menschlicher Haare.

8. Verfahren zur temporären Verformung keratinhaltiger Fasern, insbesondere menschlicher Haare, in dem
- eine pulverförmige Zusammensetzung gemäß einem der Ansprüche 1 bis 6 vor, während oder nach dem Auftragen auf die keratinhaltigen Fasern einer mechanischen Belastung ausgesetzt wird, wobei sich die pulverförmige Zusammensetzung in eine Flüssigkeit umwandelt,
- die keratinhaltigen Fasern
- vor, nach oder während des Auftragens der pulverförmigen Zusammensetzung und/oder
- vor, nach oder während der mechanischen Belastung der pulverförmigen Zusammensetzung in Form gebracht werden,
- die Form der keratinhaltigen Fasern durch die in besagte Flüssigkeit umgewandelte pulverförmige Zusammensetzung temporär fixiert wird.

## Claims

1. A powdered composition comprising core-shell particles, the shell of which contains particles of at least one hydrophobized metal oxide powder, and the liquid core of which contains, based on the weight of the core, from 20.0 wt.% to 100.0 wt.%, in particular from 50.0 wt.% to 100.0 wt.%, of at least glycerol, **characterized in that** it additionally contains at least one cationic, amphoteric or anionic setting polymer.

2. The powdered composition according to claim 1, **characterized in that** the hydrophobized metal oxide of the hydrophobized metal oxide powder is selected from at least one representative of the group comprising hydrophobized silicates, hydrophobized aluminum silicates, hydrophobized titanium dioxide and hydrophobized silicon dioxide.

3. The powdered composition according to either claim 1 or claim 2, **characterized in that** it contains hydrophobized metal oxide powder in an amount of from 0.5 wt.% to 30 wt.% based on the weight of the entire powdered composition.

4. The powdered composition according to one of claims 1 to 3, **characterized in that** the hydrophobized metal oxide powder is selected from at least one representative of the group comprising hydrophobized silicates, hydrophobized aluminum silicates, hydrophobized titanium dioxide and hydrophobized silicon dioxide, preferably Silica silylate.

5. The powdered composition according to one of claims 1 to 4, **characterized in that** at least one further solvent, in particular water, preferably in an amount of from 0 to 40 wt.%, particularly preferably from 0 to 20 wt.%, very particularly preferably from 0 to 10 wt.%, based in each case on the weight of the liquid core, is additionally contained in the powdered composition according to the invention.

6. The powdered composition according to one of claims 1 to 5, **characterized in that** it additionally contains at least one mineral salt.

7. The use of a powdered composition according to one of claims 1 to 6 for temporarily reshaping keratin-containing fibers, in particular human hair.

8. A method for temporarily reshaping keratin-containing fibers, in particular human hair, in which
- a powdered composition according to one of claims 1 to 6 is subjected to mechanical stress before, during or after application to the keratin-containing fibers, the powdered composition converting to a liquid,
- the keratin-containing fibers are shaped
- before, after or during application of the powdered composition, and/or
- before, after or while the powdered composition is subjected to mechanical stress,
- the shape of the keratin-containing fibers is temporarily fixed by means of the powdered composition that has converted to said liquid.

## Revendications

1. Composition en poudre comprenant des particules de type coeur-enveloppe, dont l'enveloppe des particules contient au moins une poudre d'oxyde métallique à traitement hydrophobe, et dont le coeur liquide contient de la glycérine à hauteur de 20,0 à 100,0 % en poids, en particulier de 50,0 à 100,0 % en poids, rapporté au poids du coeur,
**caractérisée en ce qu'**elle contient en plus au moins un polymère cationique, amphotère ou anionique durcisseur.

2. Composition en poudre selon la revendication 1, **caractérisée en ce que** l'oxyde métallique à traitement hydrophobe de la poudre d'oxyde métallique à traitement hydrophobe est choisi comme au moins un représentant du groupe constitué de silicates à traitement hydrophobe, aluminosilicates à traitement hydrophobe, dioxyde de titane à traitement hydrophobe ainsi que de dioxyde de silicium à traitement hydrophobe.

3. Composition en poudre selon la revendication 1 ou 2, **caractérisée en ce qu'**elle contient la poudre d'oxyde métallique à traitement hydrophobe à hauteur de 0,5 à 30 % en poids rapporté au poids de la composition en poudre totale.

4. Composition en poudre selon l'une des revendications 1 à 3, **caractérisée en ce que** la poudre d'oxyde métallique à traitement hydrophobe est choisie comme au moins un représentant du groupe constitué de silicates à traitement hydrophobe, aluminosilicates à traitement hydrophobe, dioxyde de titane à traitement hydrophobe ainsi que de dioxyde de silicium à traitement hydrophobe, de préférence de silylate de silice.

5. Composition en poudre selon l'une des revendications 1 à 4, **caractérisée en ce qu'**elle contient en plus au moins un autre solvant, en particulier de l'eau, préférentiellement à hauteur de 0 à 40 % en poids, particulièrement préférentiellement de 0 à 20 % en poids, tout particulièrement préférentiellement de 0 à 10 %, rapporté au poids du coeur liquide.

6. Composition en poudre selon l'une des revendications 1 à 5, **caractérisée en ce qu'**elle contient en plus au moins un sel minéral.

7. Composition en poudre selon l'une des revendications 1 à 6 pour déformer temporairement des fibres de kératine, en particulier des cheveux humains.

8. Procédé de déformation temporaire de fibres de kératine, en particulier de cheveux humains, dans lequel :
- on expose une composition en poudre selon une des revendications 1 à 6 à une sollicitation mécanique avant, pendant ou après son application sur les fibres de kératine, à l'occasion de quoi la composition en poudre se transforme en liquide,
- on met en forme les fibres de kératine :
- avant, pendant ou après l'application de la composition en poudre, et/ou
- avant, pendant ou après la sollicitation mécanique de la composition en poudre,
- la forme des fibres de kératine est temporairement fixée par la composition en poudre transformée en liquide.
